# EUROPEAN PATENT SPECIFICATION

(11) **EP 4 101 881 B1**
(45) Date of publication and mention of the grant of the patent: **19.02.2025**
(21) Application number: 21178933.4
(22) Date of filing: 11.06.2021
(51) Int. Cl.: C08J 11/16, C08J 11/12, C08J 11/18

(54) **A METHOD AND INSTALLATION FOR RECYCLING A POLYSTYRENE BASED MATERIAL CONTAINING ORGANOHALOGEN FLAME RETARDANT AND/OR FREONEN**
VERFAHREN UND EINE ANLAGE ZUM RÜCKFÜHREN EINES POLYSTYROLBASIERTEN MATERIALS MIT ORGANOHALOGENFLAMMSCHUTZMITTEL UND/ODER FREONEN IN STYROL
PROCÉDÉ ET INSTALLATION POUR LE RECYCLAGE D'UN MATÉRIAU À BASE DE POLYSTYRÈNE CONTENANT UN IGNIFUGEANT ORGANOHALOGÉNÉ ET/OU DU FRÉON

(43) Date of publication of application: 14.12.2022
(73) Proprietor: Synthos S.A., 32-600 Oswiecim (PL)
(72) Inventor: Vossebeld, Martinus Andrija, 7483 BB Haaksbergen (NL)
(74) Representative: Arnold & Siedsma

(56) References cited:
- JP-A- S 492 878
- ARANDES JOSÉ M ET AL: "Thermal recycling of polystyrene and polystyrene-butadiene dissolved in a light cycle oil", JOURNAL OF ANALYTICAL AND APPLIED PYROLYSIS, vol. 70, no. 2, 1 December 2003 (2003-12-01), NL, pages 747 - 760, XP055875237, ISSN: 0165-2370, DOI: 10.1016/S0165-2370(03)00056-1
- JUNG SU-HWA ET AL: "Fast pyrolysis of a waste fraction of high impact polystyrene (HIPS) containing brominated flame retardants in a fluidized bed reactor: The effects of various Ca-based additives (CaO, Ca(OH)2and oyster shells) on the removal of bromine", FUEL, vol. 95, 11 January 1974 (1974-01-11), pages 514 - 520, XP028889831, ISSN: 0016-2361, DOI: 10.1016/J.FUEL.2011.11.048

## Description

### BACKGROUND OF THE INVENTION

Polystyrene based materials comprise polystyrene, expanded polystyrene (EPS), extruded polystyrene (XPS), such as used in packaging foils and containers, styrene-butadiene rubber (SBR), and acrylonitrile-butadiene-styrene (ABS). These materials may originate from renovation construction elements comprising a (expanded) polystyrene core and a polyolefin foil, or from electric and electronic equipment which nowadays have a short product life cycle.

These polystyrene based materials comprise flame retardants which are activated under ignition conditions, such as fire or heat, and are intended to prevent or slow the further development of ignition or fire breakthrough. When recycling polystyrene containing a flame retardant it is necessary that the flame retardant is removed in a manner such that it will not enter the environment. This holds in particular for organohalogen flame retardants, examples of which are organobromo flame retardants, such as hexabromocyclododecane (HBCD), tetrabromobisphenol A (2,3-Dibromopropyl)ether (BDDP or FR-720), tris(tribromophenoxy) triazine (FR-245), Tetrabromobisphenol-A-bis(2,3-dibromo-2-methylpropylether, 1,1'-(isopropylidene)bis[3,5-dibromo-4-(2,3-dibromo-2-methylpropoxy)benzene] (TBBPA-DBMPE or AP 1300 SF), a block copolymer of polystyrene and brominated polybutadiene (FR-122P), organochloro flame retardants, such as Tris(1-chloro-2-propyl) phosphate (TCPP) and polyvinylchloride (PVC), and organofluoro flame retardant tetradecafluorohexane (TDFH). Expanded polystyrene based materials comprise freonen used as expansion agent. It are organofluoro compounds, such as hydrochlorofluorocarbons (HCFC, such as R-22, R-12 and R134a).

Several processes are known from the art for the removal of bromine from styrene polymers containing bromated flame retardant. Grause et al., in Polymer Degradation and Stability, 112, pages 86-93, 2015, uses a solution of NaOH in ethylene glycol for removing decabromodiphenenyl ethane (DBPE) from high-impact polystyrene. The debromination ratio was 42% at 190°C. The ratio was decreased to about 0.02 wt% by a mechanical treatment using a ball mill reactor. Ukisu in Chemosphere, 179, pages 179-184, 2017, studied catalytic debromination of HBCD using a silica-supported palladium catalyst in a solution of 2-propanol/methanol containing dissolved NaOH at 35°C. The reaction product yielded 92% for bromine-free products. Evangelopoulos et al., in Waste Management, 94, pages 165-171, 2019, studied the removal of tetrabromobisphenol (TBBPA) from brominated plastics, modem Wi-Fi plastics, and printed circuit boards using solvent extraction with isopropanol or toluene followed by pyrolysis. Reportedly the degree of removal of bromated compounds was relatively low. Wang et al., in J. Hazardous Materials, 205-206, pages 156-163, (2012) studied the debromination of ABS containing TBBPA. Under supercritical conditions (400 °C, 9 to 19 MPa) water, methanol, isopropanol, and acetone have been used. Water showed highest debromination efficacy (97.6%). Alkali were added to supercritical isopropanol, and salts formed dissolved in the supercritical alcohol and precipitated under ambient conditions. NaOH and KOH use resulted in a bromine yield in the treated product of about 5 wt%. Dement'ev et al/. in J. Anal. Appl. Pyrolysis, 142, pages 1-10, 2019, studied the thermal depolymerization of polystyrene in highly aromatic hydrocarbons. At 500°C the styrene yield was 84.4% at a polymer conversion of 93.8%. JUNG SU-HWA ET AL describe in "Fast pyrolysis of a waste fraction of high impact polystyrene (HIPS) containing brominated flame retardants in a fluidized bed reactor: The effects of various Ca-based additives (CaO, Ca(OH)2 and oyster shells) on the removal of bromine",FUEL, vol. 95, 11 January 1974, pages 514-520, XP028889831 a method/ installation for the pyrolysis of a waste fraction of high impact polystyrene (HIPS) containing brominated flame retardants in a fluidized bed reactor; the effects of various Ca-based additives (CaO, Ca(OH)2 and oyster shells) on the removal of bromine.

The present invention has for its object to recycle polystyrene based material providing styrene of high purity that can be used as virgin or added styrene monomer and other valuable products for sale or reuse in the method, while generating minimal by-products and no formation of halogenated hydrocarbons, such as PCBs and PBBs. Still the dehalogenation treatment is carried out under moderate conditions (mild temperature and atmospheric pressure) in a basic organic environment without a catalyst and common reaction vessel. Thus, the invention aims at providing a method and installation to carry out the method, in which all reactions are carried out in a liquid phase using preferably or predominantly solvents evolving when carrying out the method. The materials used and reaction conditions applied are such that it is not necessary to operate the compaction part of the installation, particularly on location, under ATEX conditions as explosive atmospheres will not occur. And as to transportation of materials and intermediate products (such as compacted polystyrene), they fall in the lowest classification of the ADR (Accord européen relatif au transport international des marchandises Dangereuses par Route).

It is important to appreciate that the present invention is able to recycle a variety of different polystyrene based materials, such as packaging as yogurt cups or bowls. Particularly, for expanded, foamed, and extruded materials, the compaction is not thermal and/or chemical compaction.

### SUMMARY OF THE INVENTION

According to this objective, the invention provides a method for recycling a polystyrene based material containing organohalogen flame retardant and/or freonen, comprising the steps of:
(i) dissolving the polystyrene based material in a high-boiling, apolar, organic reaction solvent;
(ii) heating the polystyrene contained in the reaction solvent to a temperature so as to release halogen compound from the flame retardant and/or freonen;
(iii) contacting released halogen compound with a base so as to form a halogen salt;
(iv) removing the halogen salt;
(v) pyrolyzing polystyrene contained in the reaction solvent at a temperature so as to depolymerize polystyrene; and
(vi) distilling the depolymerized mixture into at least a styrene fraction.

The method of the invention comprises the dissolution of polystyrene containing an organohalogen flame retardant and/or freonen, and possible foreign plastics in the high-boiling reaction solvent from which solution halogen present in the flame retardant compound is released as halogen compound. The halogen compound is captured by a base and removed as the halogen salt. The dehalogenated polystyrene still present in reaction solvent is then pyrolyzed to depolymerize it into at least styrene. The styrene contains minimal amounts of remaining organohalogen flame retardant or freonen and any halogen compounds and suitable for use as virgin monomer or additional styrene monomer. The use of the high-boiling reaction solvent in all method steps allows for operation under non-ATEX conditions during compaction, particularly on location, and handling and routing of products and intermediate products under lowest ADR conditions.

In one embodiment of the invention, the halogen compound released from the flame retardant may be recuperated from the reaction solvent and separate from the solvent contacted with a base in order to form a halogen salt. Under these circumstances practically any base able to form such halogen salt may be used. In a preferred embodiment, the base is added to the reaction solvent so that halogen compound released in step (ii), is contacted in step (iii) with the base so that the halogen salt is formed in the reaction solvent. This means that the base is present in the reaction solvent at the time during heating step that halogen compound is released from the organohalogen containing flame retardant or freon. The released halogen compound is there and then captured and converted into a halogen salt which is easily removed as solid from the reaction solvent. Accordingly, the halogen compound such as in the form a halogen acid which is a corrosive agent, is not or only shortly present in the reaction solvent, so that there is no need for using corrosion resistant material for the installation equipment. Moreover, under the heated conditions of step (ii) and during the subsequent pyrolysis step (v) no halogen compound is present and thereby any side-reactions of halogen compound with solvent or depolymerization products are avoided.

It is particularly preferred that the base added to the apolar, organic reaction solvent is dissolved therein, so that the reaction with halogen proceeds optimally in the liquid phase, which makes the removal of the halogen salt easy. And avoids any side stream such as water and polar (organic) solvent containing the base. Therefore, the base used for forming the halogen salt in the reaction solvent is selected from the group comprises sodium diamine, potassium tert-butoxide (KOtBu), sodium bis(trimethylsilyl)amide, and P(CH3NCH2CH2)3N. These selected bases are soluble in the reaction solvent. Small volatiles formed may be removed and neutralized.

Essentially a versality of different polystyrene based materials may be used, such as polystyrene, high impact polystyrene (HIPS), expanded polystyrene (EPS), extruded polystyrene (XPS), styrene-butadiene rubber (SBR), acrylonitrile-butadiene-styrene (ABS), and polymethylmethacrylaat (PMMA). Several of these materials comprise additional monomers (or polymers) which will have no negative on the performance of the method. This holds for polystyrene based materials comprising up to about 10 wt% foreign polymer, such as polyethylene, polypropylene, and/or polyvinylchloride. Examples used are Piocelan a polypropylene-compounded polystyrenic foamed resin and a polyethylene-compounded polystyrenic foamed resin. Also these foreign polymers will not hamper the method and ultimately will end up in a rest-product stream.

The dehalogenation of the flame retardant and/or freonen in step (ii) is generally carried out by heating the reaction solvent containing polystyrene to a temperature in the range of about 150°C to about 350°C, preferably about 180°C° to about 300°C. The pressure corresponding to these temperatures varies between about 1 bar to about 20 bar, preferably about 1 bar to about 15 bar, such as 1 to 12 bar.

The dehalogenation reaction starts at elevated temperatures, and the higher the temperature the shorter the time to completion of the reaction. Reaction time for the dehalogenation is generally between 2 to 120 min, but halogenation is normally almost complete between 5 to 70 min, such as between 10 and 60 min.

The method of the invention is particularly intended for the recycling of expanded, foamed or extruded polystyrene or mixtures of polystyrene and expanded, foamed or extruded polystyrene, and of their related polystyrene based materials. But due to the relative low density of expanded polystyrene their application is the basic method of the invention is less practical. Therefore, the invention provides in a generally preferred embodiment an additional pre-step in the method in which the expanded polystyrene is compacted and its density substantially increased. In addition, the compacted polystyrene is converted into a semi-solid form stiff material or two-phase system dependent on the type and amount of compaction solvent used. But they can be easily handled and are less or not sticky. But in order to compact the expanded polystyrene properly or optimally it is required to use a solvent or solvent mixture different in properties of the reaction solvent. Thus, the invention provides a pre-step which is a step of compacting expanded polystyrene in a compaction solvent. With this compaction solvent the expanded polystyrene forms a semi-solid material or a two-phase system comprising a semi-solid (dough-like) phase of the polystyrene. This compacted form-stiff polystyrene is separated from the liquid phase by any suitable technique.

This two-phase system or the compacted polystyrene may be subjected to the heating step for dehalogenation. But it is preferred to add the reaction solvent or that the compaction solvent is at least partly replaced by the reaction solvent. And after this replacement the compacted polystyrene enters step (i) of the method of the invention.

At this point it is noted that the compaction pre-step is not necessarily to be carried out in time and/or location directly prior to step (i) or step (ii) of the method of the invention. It is equally possible while maintaining all benefits of the invention to perform the pre-step earlier in time and/or even at a different location. Particularly, if the availability and/or presence of polystyrene material to be recycled by time is less than the processing rate of the method. Then expanded polystyrene to be recycled is advantageously compacted at one or more remote locations and transported to a central processing site where the compacted polystyrene of different locations, of different types and/or of different sources is processed. All transport and processing proceeds at general and common conditions as working under ATEX conditions is not required and two-phase system and the compacted polystyrene are materials which fall in the lowest ADR classification.

One of the important finding of the present invention is that the pyrolyzation mixture obtained after pyrolyzation in step (v) comprises an aromatic and/or aliphatic fractions that can be obtained by distillation in step (vi), and have properties of compacting expanded polystyrene present in the polystyrene based material and/or properties of the reaction solvent. They are method-own solvent and distillation fractions.

For the compaction a distilled fraction has such a high boiling point that compaction related processing is carried out at least 10°C below the flashpoint so that processing is not to be carried out under ATEX conditions. When using for compaction only the aromatic fraction then compaction results in the formation of a polystyrene solution with the consistency of a syrup. Using a mixture of the aromatic fraction and the aliphatic mixture compaction results in the formation of a two-phase system with the consistency of a paste or of a form stiff product with the consistency like camembert cheese. The more aliphatic fraction is used the more the consistency becomes stiffer and the polymer density increased. under liquid conditions. Therefore the method of the invention applies at least partly as the compaction solvent a mixture of an aliphatic solvent and an aromatic compaction solvent, such as a high-boiling distillate fraction obtainable in step (vi) preferably a styrene dimer fraction boiling at about 280°C to about 320°C, or boiling at about 150°C to about 270°C, such as about 180°C to about 250°C at about 10 to 30 mbar absolute pressure. Alternatively, the dimer fraction may contain up to C16 and has a boiling point of about 290°C to 305°C.The aromatic compaction solvent and an aliphatic compaction solvent, are preferably a fraction obtainable in the distillation of step (vi).

The volume ratio of the aromatic solvent and the aliphatic solvent is selected dependent on the composition of the (expanded, foamed or extruded) polystyrene based material. But the percentage of flame retardant present is generally relatively small, so that the solvent mixture is generally present in excess. But as the Some flame retardants such as HBCD dissolves only slightly in the aliphatic solvent and good in the aromatic solvent, whereas any present polyethylene and/or polypropylene dissolves better in the aliphatic solvent. Hence, the volume ratio of the aromatic compaction solvent and the aliphatic compaction solvent selected dependent on the type of polystyrene material to the recycled and in regard of the consistency of the compacted mass. For instance at >20% aromatic solvent becomes the compacted mass form stiff. Accordingly, the volume ratio may be in the range of about 30:70 (paste consistency), preferably about 20:80, more preferable about 10:90 (stiff consistency).

Another the important finding of the present invention is that the pyrolyzation mixture obtained after pyrolyzation in step comprises a fraction of that has properties of dissolving the dehalogenated polystyrene and of having a such high boiling point that both the dehalogenation of step (ii) and the pyrolyzation of step (v) can be performed under liquid conditions. This reaction solvent is also a system-own product, and any degradation of it would result in styrene and styrene oligomers which can be used. Therefore the method of the invention applies at least partly a high-boiling apolar organic reaction solvent having a vapor pressure of less than about 10 bar at a temperature in the range of about 250°C to about 450°C. This reaction solvent is preferably a high-boiling distillate fraction obtainable in step (vi), preferably a styrene trimer fraction boiling at about 380°C to about 420°C. Alternatively, the trimer fraction may contain up to C24 and has a boiling point of about 400°C

Due to the difference in composition and properties of the compaction solvent and the reaction solvent, is preferred that the compaction solvent is replaced at least partly by the reaction solvent prior to the dehalogenation step (ii), and preferably the compaction solvent is recycled to the compaction step.

When preferably the reaction of the released halogen compound in step (ii) with the base is carried out in the reaction solvent when release of halogen is eminent, then it is preferred that the removal of the halogen salt is carried out on stream from the reaction solvent by solid liquid separation such as filtration, centrifugation, (hydro) cyclonic separation, at a salt particle size of less than about 20 µm, preferably less than about 20 µm, such as less than 1µm, or less than 0.4 µm. Such salt removal has the additional advantage that any sand or other particulate material present in the styrene based material to be recycled is removed which is beneficial to processing and reduces the risk for installation damage due to abrasion.

For the practical and sufficient removal of any organohalogen flame retardant or freonen it is preferred that the base is present in slight excess, such that the molar ratio of base to the organohalogen flame retardant and/or freonen is about 1.1 to about 3, such as about 1.5 to about 2.6, or 1.1 to 1.5.

As referred to above, the pyrolysis step (v) is carried out under more stringent conditions than the dehalogenation step (ii). Practically the pyrolysis of step (v) is carried out at atmospheric pressure at a temperature about 400°C to about 550°C, preferably at about 500°C to about 520°C, or at higher pressure and at correspondingly lower temperatures. Preferably the pressure is about 1 bar to 3 bar.

The pyrolysis step (v) may be carried out in a meltbed reactor. This is a reactor of common and simple design and can be applied because the pyrolysis is carried out under liquid conditions due to the use of the high-boiling reaction solvent. This liquid state pyrolysis is optimal for heat transfer and substantially avoids pyrolysis to solid materials such as soot.

Processing the method of the invention optimally has the beneficial result that the removal of organohalogen flame retardant and/or freonen present in the polystyrene based material is at least 90%, preferably at least 95%, more preferably at least 99%. Accordingly, the styrene product comprises flame retardant and/or freonen in an amount of less than 15 ppm, preferably less than 10 ppm, such as less than 5 ppm.

Another aspect of the invention relates to an installation for recycling a polystyrene based material containing an organohalogen flame retardant and/or freonen, such as defined and discussed hereinbefore. Such installation comprises:
- a dissolution unit (a) for dissolving the polystyrene based material in a high-boiling, apolar, organic reaction solvent or in a mixture with a high boiling aliphatic reaction solvent;
- a heating unit (b) for heating the polystyrene contained in the reaction solvent to a temperature so as to release halogen compound from the flame retardant and/or freonen;
- a contacting unit (c) for contacting released halogen compound with a base so as to form a halogen salt;
- a halogen salt removing unit (d) for removing the halogen salt from the reaction solvent;
- a pyrolyzation unit (e) for pyrolyzing the polystyrene in the reaction solvent at a temperature so as to depolymerize polystyrene; and
- a distillation unit (f) for distilling the depolymerized mixture into at least a styrene fraction.

As discussed above the installation is suitable for compacting expanded polystyrene and thereto additionally comprises a compaction unit for compacting expanded polystyrene in a compaction solvent, and for preferably replacing the compaction solvent by the reaction solvent.

It is also beneficial that the reaction solvent used for the dehalogenation and pyrolysis at least partly is generated when carrying out the method of the invention, To that extent the installation comprises a distillation unit (f) which distils the depolymerized mixture to a styrene trimer fraction which is recycled and used at least in apart as reaction solvent. For the same reasons, the distillation unit (f) distils the depolymerized mixture to a styrene dimer fraction which is recycled and is used at least in apart as compaction solvent for expanded polystyrene.

As discussed above, the pyrolysis is carried out under liquid conditions due to the use of the high-boiling solvent. This allows for the use of a meltbed reactor. This is a reactor of common and simple design and can be applied because this liquid state pyrolysis is optimal for heat transfer and substantially avoids pyrolysis to solid materials such as soot. Thus, the pyrolyzation unit (e) comprises a meltbed reactor. Additional advantages of the use of a meltbed reactor will be discussed in the description below.

The method and the installation of the invention are suitable for recycling a variety of different polystyrene based materials comprising a great number of different flame retardant and/or freonen. Examples of the polystyrene based materials are polystyrene, expanded polystyrene (EPS), extruded polystyrene (XPS), such as used in packaging foils and containers, styrene-butadiene rubber (SBR), acrylonitrile-butadiene-styrene (ABS). Examples of the organohalogen flame retardants are organobromo flame retardants, such as hexabromocyclododecane (HBCD), tetrabromobisphenol A (2,3-Dibromopropyl)ether (BDDP or FR-720), tris(tribromophenoxy) triazine (FR-245), Tetrabromobisphenol-A-bis(2,3-dibromo-2-methylpropylether, 1,1'-(isopropylidene)bis[3,5-dibromo-4-(2,3-dibromo-2-methylpropoxy)benzene] (TBBPA-DBMPE or AP 1300 SF), a block copolymer of polystyrene and brominated polybutadiene (FR-122P), organochloro flame retardants, such as Tris(1-chloro-2-propyl) phosphate (TCPP) and polyvinylchloride (PVC), and organofluoro flame retardant tetradecafluorohexane (TDFH). Expanded polystyrene based materials comprise freonen used as expansion agent. These are organofluoro compounds, such as hydrochlorofluorocarbons (HCFC, such as R-22, R-12 and R134a).

### DETAILS OF THE INVENTION

Mentioned and other characteristic and advantages of the method and installation of the present invention will become apparent from the description given hereafter which is considered to be given for information purposes only and not to limit the invention to any extent. In this respect reference is made to the annexed figures wherein:
Figure 1 is a diagram of the basic process and installation of the present invention comprising a removal step or unit for removing flame retardant and/or freonen from the polystyrene based material, a pyrolysis step or unit, and a distillation step or unit for providing by distillation styrene;
Figure 2 is a diagram of the process and installation of the present invention comprising in addition to the process and installation of Figure 1 a compaction step or unit for compacting expanded polystyrene, and a step or unit for replacing compaction solvent by reaction solvent which both are preferably recycled;
Figure 3 is a process flow diagram showing more in detail the compaction unit;
Figure 4 is a process flow diagram of an alternative compaction unit;
Figure 5 is a process flow diagram of a more detailed installation of the invention for carrying out the process of the invention;
Figure 6 is in more detail the unit for replacing at least in part of the compaction solvent by the reaction solvent;
Figure 7 is in more detail the unit for purifying the compaction solvent;
Figure 8 is in more detail the heating or dehalogenation unit;
Figure 9 is in more detail the pyrolysis unit;
Figure 10 is in more detail a quencher unit for reducing the solvent temperature after the pyrolysis;
Figure 11 is in more detail the distillation unit for providing the styrene, and distillation fraction for the compaction solvent and reaction solvent;
Figure 12 a stripper unit for purifying the compaction solvent;
Figure 13 in side view a meltbed reactor used in the process and installation of the invention; and
Figure 13A a cross-section over line A-A of Figure 13.

### EXAMPLES

### Example 1: compaction of expanded polystyrene

25 gr expanded polystyrene is added to 50 gram compaction solvent is added.

The compaction solvent comprises about 20wt%, aromatic hydrocarbons with a boiling point of about 300 °C, and about 80 wt% aliphatic hydrocarbons with a boiling point of 310°C. Compaction was carried out at about 60 to 70 °C.

After dissolution in the compaction solvent a relatively hard form-stable product is formed. This high-load polystyrene product has a solid content of about 60%.

### Example 2: compaction of expanded polystyrene based material comprising polyethylene and/or polypropylene

An expanded polystyrene product comprising 24,75 gr expanded polystyrene (95%) and 1,25 gr expanded polyethylene (5%) is added to 50 gram compaction solvent. The compaction solvent comprises about 20 wt% aromatic hydrocarbons with a boiling point of about 300 °C, and 80 wt% aliphatic hydrocarbons with a boiling point of about 300 °C. The compaction temperature is about 85 °C.

Expanded polyethylene also compacts as expanded polystyrene but a wax-like homogenic end product is obtained . The solids content is about 66%. The expanded polystyrene comprises expanded polyethylene in only a small concentration, therefore expanded polyethylene can be processed in the same manner as expanded polystyrene.

### Example 3: compaction of expanded polystyrene based material comprising polyethylene and/or polypropylene

4.3 gr expanded polystyrene based material comprising about 60 wt% polystyrene, 35 wt% foamed polypropylene, and 5 wt% foamed polyethylene was added to 65 ml of a compaction solvent mixture comprising 40 vol% aromatic hydrocarbon with a boiling point in the range of about 290 - 305 °C (Solvesso 150ND) and 60 vol% aliphatic hydrocarbon with a boiling point of about 187 - 216 °C (Vasil 60). The density of the compaction solvent solution obtained was 67 gr/l. The compaction was carried out at a temperature of 130 °C.

The expanded polyethylene and expanded polypropylene defoamed in a similar manner as expanded polystyrene. But a jelly like end product is obtained. Thus expanded polystyrene comprise some foreign polyethylene and/or polypropylene can be compacted but at higher compaction temperatures.

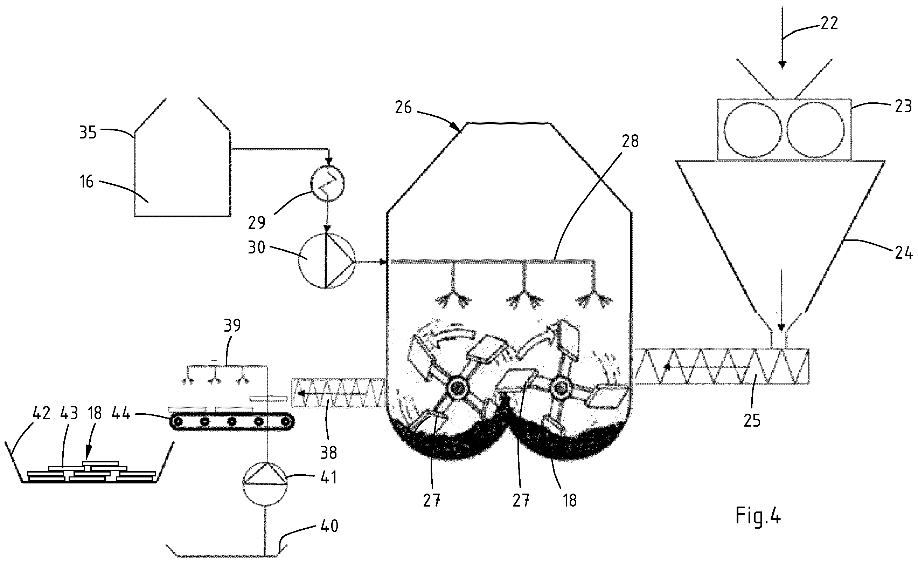

Due to the jelly like consistency of the end product a once-through-put-away concept (according to Fig. 4) may be applied in which the mixture after being saturated with defoamed expanded polystyrene, expanded polypropylene and some expanded polyethylene may be stored as briquets until further processing.

### Examples 4: dehalogenation of polystyrene

Polystyrene comprising 1 wt% flame retardant was dissolved in Solvesso 150 ND^{™} (boiling point: 183°C to 194°C, for ExxonMobil Chemical Series) as aromatic reaction solvent. A base was added to the reaction solvent at a given organohalogen to base molar ratio. The debromination was carried out at a given temperature. The conversion at the given temperature for about 60 min, was calculated as 1-(mass recovered oil * [Br] in oil)/mass Br added to the reactor).

| Example | Flame retardant | Base | Ratio | Temperature (°C) | Conversion (%) |
|---|---|---|---|---|---|
| 3a | HBCD | NaNH2 | 1 | 180 | 80 |
| 3b | HBCB | NaNH2 | 2.6 | 180 | 88 |
| 3c | HBCD | NaNH2 | 1 | 250 | 87 |
| 3d | HBCD | NaNH2 | 1.5 | 250 | 93 |
| 3e | HBCD | NaNH2 | 2 | 250 | 97 |
| 3f | HBCD | NaNH2 | 2.6 | 250 | 98 |
| 3g | HBCD | NaNH2 | 1 | 300 | 92 |
| 3h | HBCD | NaNH2 | 2.6 | 300 | 98 |
| 3j | (P)VC | NaNH2 | 2.6 | 250 | 100 |
| 3l | FR-720 | NaNH2 | 2.6 | 250 | 95 |
| 3m | FR-245 | NaNH2 | 2.6 | 250 | 98 |
| 3n | AP1300SF | NaNH2 | 2.6 | 250 | 97 |
| 30 | Tris Chloro | NaNH2 | 2.6 | 250 | 100 |
| 3p | HBCD | KOtBu | 2.6 | 250 | 100 |
| 3q | FR-245 | KOtBu | 2.6 | 250 | 100 |
| 3r | FR-720 | KOtBu | 2.6 | 250 | 100 |
| 3s | AP1300SF | KOtBu | 2.6 | 250 | 100 |

### Examples 5: dehalogenation of polystyrene with trimer fraction

Polystyrene comprising HBCD flame retardant was dissolved in a styrene trimer fraction used as aromatic solvent. NaNH₂ base was added to the reaction solvent at a given organohalogen to base molar ratio of 2.6. The debromination was carried out at 250°C. The conversion after 60 min at the reaction temperature was calculated as 1-(mass recovered oil * [Br] in oil)/mass Br added to the reactor) and was about 99%.

### Example 6: dehalogenation of expanded polystyrene with trimer fraction

Expanded polystyrene containing 0.71 wt% HBCD was subjected to dehalogenation using a mixture of styrene dimer and styrene trimer (obtained by pyrolyzation of polystyrene beads dissolved in Solvesso 150 ND^{™}, at 350°C, and removal of styrene monomer by fractional distillation at 100°C at 100 mbar) as compaction solvent. The reactor loading was 10 wt% EPS, which corresponds to a flame retardant loading of 0.1 wt%. NaNH2 was used as base and the base/Br-ratio was 2.6. The reaction temperature was 250°C, and the reaction time 60 min. The dehalogenation conversion was 98% for the EPS (HBCD) sample.

### Example 7: pyrolyzation of compacted polystyrene

The compacted polystyrene obtained in Example 5 was subjected to pyrolyzation for the production of in particular styrene by depolymerization of polystyrene. The liquid mixture of polystyrene and styrene trimer (obtained after distillation of the dimer/trimer mixture of Example 6 at 250°C at 20 mbar absolute pressure) was fed to a pyrolyzation reactor and subjected to pyrolyzation at a temperature of 500°C for about 5 to 10 min at atmospheric pressure. The pyrolyzed oily liquid obtained was cooled by quenching with cooled reaction solvent to a temperature of about 160°C. After removal of any solids, such as char and soot, the cooled liquid mixture was subjected to a stripping operation for obtaining a styrene product stream of which styrene as the major component as top distillate. Obtained are further a middle distillate fraction and a bottom fraction.

The styrene product stream was subject to a four-stage distillation units for distilling off an aliphatic fraction that may be used as aliphatic solvent, an light aromatics fraction, and heavy ends as bottom fraction. The top fraction is styrene which boils at between about 145°C, and has a purity of at least 98% and contains less than 7 ppm flame retardant.

The middle distillate fraction was further distilled and provided light nafta and a fraction boiling at between about 280°C and 320°C, and represents the dimer fraction used as compaction solvent. The bottom distillate fraction boils at about 380 °C to 420°C and represent the trimer fraction used as reaction solvent.

### DESCRIPTION OF THE INVENTIVE METHOD AND INSTALLATION

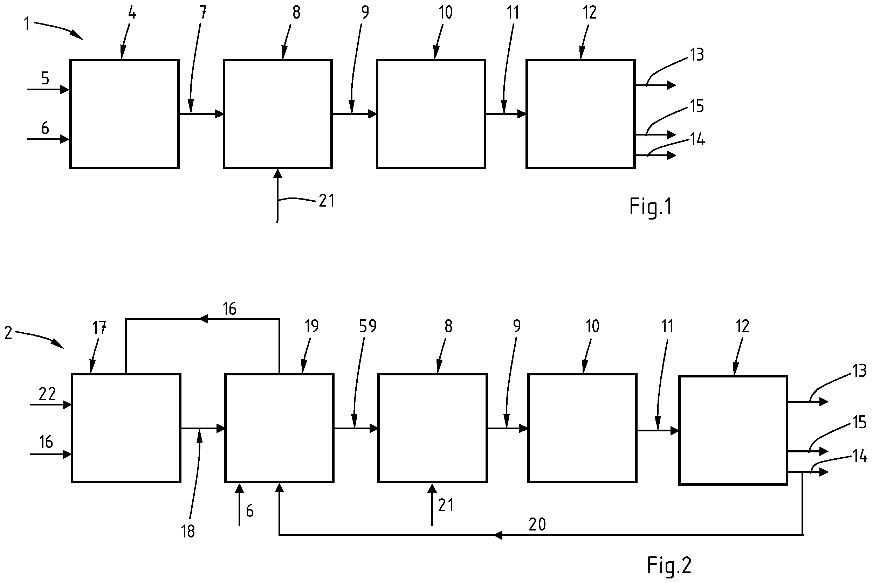

Figure 1 shows a general process flow diagram of a method and installation 1 of the present invention. The installation 1 comprises a dissolution unit 4 to which are added polystyrene based material 5 containing organohalogen flame retardant for dissolution or dispersion in added reaction solvent 6. The mixture 7 containing polystyrene in the reaction solvent 6 is added to a heating unit 8. In the heating unit 8 the polystyrene present in the reaction solvent 6 is heated to a temperature for a time period sufficient to release halogen compound from the flame retardant. The heating temperature is preferably about 180°C to about 300°C, such as 250°C for about 10 min to 120 min, preferably about 45 min to about 90 min, such as 60 min.

Released halogen compound is converted into a halogen salt with base 21 added. The halogen salt is removed with a hydrocyclone or by filtration. Polystyrene 9 devoid of halogen and contained in the reaction solvent is added to a pyrolyzation unit 10 in which polystyrene 9 is pyrolyzed and depolymerized into styrene 13, valuable products 14, and pyrolyzed solids such as soot 15. The pyrolyzation is carried out at about 400°C to about 600°C, or at 425°C to about 550°C, such as 450°C to about 500°C.

The pyrolyzation mixture 11 in the reaction solvent is added to a distillation unit 12, where in styrene 13 and valuable products 14 are distilled off. Other distillation liquids and gas may be used for generation energy. The pyrolyzation solids 15 are removed.

Figure 2 shows another general process flow diagram of a method and installation 2 of the invention. In comparison to the installation 1 of Figure 1, installation 2 additionally comprises a compaction unit 17 in which expanded polystyrene based material 22 is compacted with compaction solvent 16. The mixture of compacted polystyrene 18 contained in the compaction solvent 16 is added to a separation unit 19 in which the compacted solvent is at least partly replaced by the reaction solvent 6. The replaced compaction solvent 16 is recycled to the compaction unit 17. The reaction solvent 6 added to the separation unit 19 may be fresh reaction solvent 6 and/or may be recycled reaction solvent 20 obtained as a valuable product 14 in the distillation unit 12.

The mixture of compacted polystyrene and compaction solvent may have the form of a two-phase system with a semi-solid polystyrene phase and a liquid phase, or a single semi-solid phase which can be transported with common means, such as a pump. The type of the compacted form depends on the amount of compaction solvent added and the type of expanded polystyrene based material, and is as desired or needed. If this material also comprises polyethylene and/or polypropylene then another valuable distillation product 14 comprises an aliphatic solvent, a mixture of the aliphatic solvent with the reaction of compaction solvent improves the dissolution of the flame retardant and/or freonen contained in the polystyrene based material.

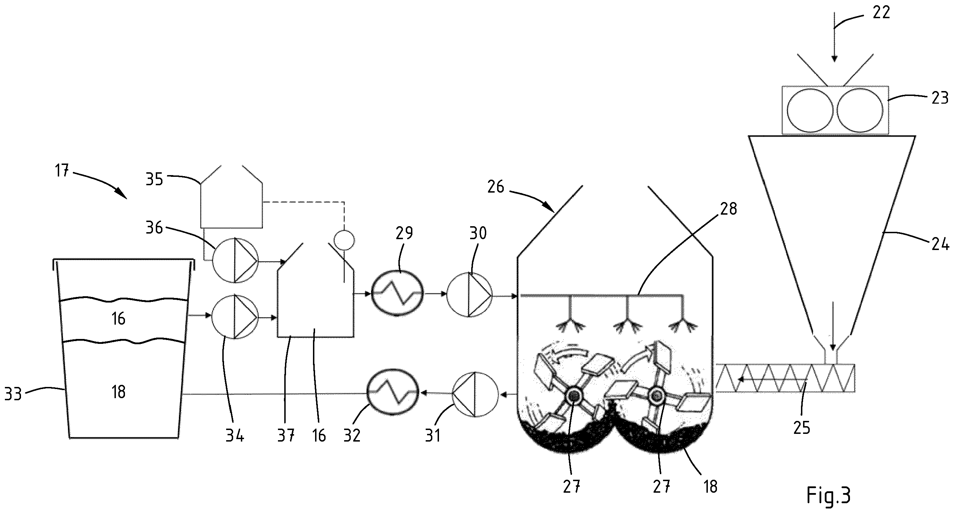

Figure 3 shows more in detail a compaction unit 17 according to the invention. Expanded polystyrene 22 is added to a storage hopper 24 and transported with a conveyor screw 25 to a compaction mill 26. Here the expanded polystyrene is compacted with compaction fluid 16 supplied by sprayer unit 28 and impacted with the expanded polystyrene with rotating compaction arms 27. The compacted polystyrene 18 formed is transported via a pump 31 and cooling unit 32 to a storage tank 33. The tank 33 comprises a two-phase system comprising a phase of semi-solid compacted polystyrene 18 and a phase of liquid compaction fluid. That surplus of compaction fluid 16 may be recycled via pump 34 to compaction fluid tank 37 also provided with fresh compaction fluid from storage 35 via pump 36.

The compacted polystyrene 18 collected in tank 33 may be directly added to the heating unit 17 or first transported from a remote location to a central installation 1-3.

Figure 4 shows in more detail another compaction unit 17 of the present invention (same reference numbers refer to same elements of the compaction unit 17 described in Figure 3. Different from the compaction unit 17 of Figure 3, here the compaction unit operates under the once-through-put-away principle by which this compaction unit 17 is adapted to compact expanded, foamed or extruded polystyrene that contains higher amounts of polyethylene and/or polypropylene, such as amounts up to about 5 to about 10 wt%. This implies that compacted polystyrene 18 is transported with an extrusion conveyor onto a porous conveyor belt 44, and spray cooled with cooling liquid 39 supplied with pump 41 and collected on a dripping tray 40. Briquette 43 of compacted polystyrene are collected in a bin 42. Compaction solvent 16 leaving the compaction unit 17 with the briquette 43 is compensated for and supplied from tank 35 and heater 29 and pump 30.

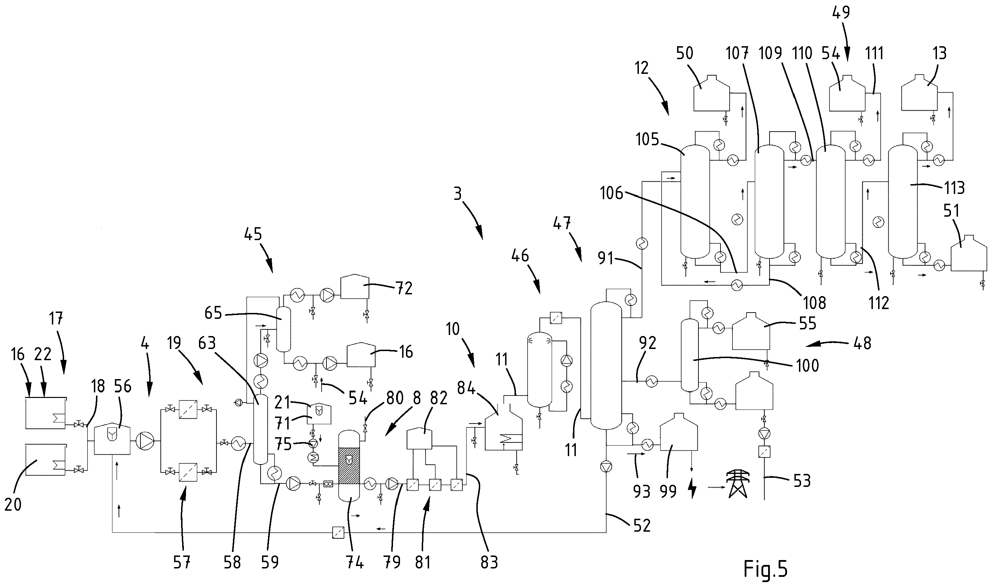

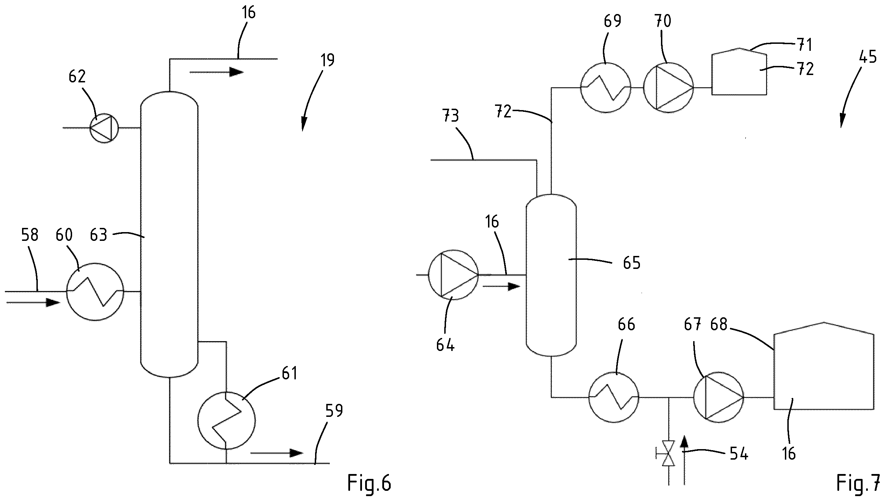

Figure 5 shows a non-limiting example of a process flow diagram of another installation 3 of the invention for recycling polystyrene based material 5 into styrene 13 and a trimer fraction 52, aliphatic solvent 50, light aromatics 54, and light nafta 55. Produced is also a dimer fraction 53 to be used as compaction solvent when expanded polystyrene is processed. Major unit operations will be discussed and details thereof with be described in the Figures 6-13.

The installation 3 comprises a compaction unit 17 for compaction of expanded polystyrene material 22 in compaction solvent 16, as described in relation to Figures 3 and 4. The volume ratio compaction solvent 16 to expanded polystyrene 22 is about 1:1. Compacted polystyrene 18 is fed to the dissolution unit 4 comprising a mixing tank 56, and dissolved in supplied reaction solvent 20 and/or trimer fraction 52 (optionally in admixture with aliphatic solvent 50).

The dissolved polystyrene 7 is filtered and enters separation unit 19 comprising a solvent reclaim section 45, for separating compaction solvent 16. Polystyrene contained in reaction solvent 6 is transported into a heating unit 8 for the dehalogenation of flame retardant and/or freonen. Released halogen compound reacts with base 21 present in the reaction solvent and/or added in the form of a mixture with aliphatic/aromatic solvent mixture from tank 71 via pump 75, and halogen salt formed is filtered off. Polystyrene 9 devoid of halogen and present in reaction solvent 6 is in the form of mixture 83 subjected to pyrolyzation in pyrolyzation unit 10 at a temperature of about 510°C. The pyrolyzed mixture is cooled in a quencher unit 46 with cooled reaction solvent, i.e., in the trimer fraction 52.

After removal of soot and char by filtration the filtered pyrolyzed mixture 11 is subjected in a distillation unit 12 to distillation sections 48 and 48 for providing pure styrene 13, dimer fraction 53 to be used a compaction solvent 16, trimer fraction to be used as reaction solvent 6, aliphatic solvent 50 to be used in admixture with compaction solvent 16, and light aromatics 54 to be used in admixture with reaction solvent 6. Other product streams may be used for generating energy and/or heating.

Figure 6 shows in more detail de separation unit 19. A liquid mixture 58 of about 1 part polystyrene, about 1 part compaction solvent 16 and about 2 parts reaction solvent 6 is heated with heater 60 and enters a flash drum 63 provided with a vacuum pump 62 for flashing the mixture 58. Lower boiling compaction solvent 16 leaves the flash drum over its top and is conveyed to the solvent reclaim section 45. A liquid mixture 59 of about 2 parts higher boiling reaction solvent 6 and about 1 part dissolved polystyrene is bottomed and forwarded to the heating section 8. Temperature of the mixture is controlled with a reboiler 61 as to maintain polystyrene in liquid state.

Figure 7 shows in more detail the compaction solvent reclaiming section 45. The compaction solvent 16 added over the pump 64 is stripped in stripper 65 and after cooling in condenser 66 supplied with pump 67 to storage tank 68. The stripped light fraction 72 is cooled in condenser 69 and applied via pump 70 to storage tank 71. The stripper may be operated at subatmospheric pressure by connection of a vacuum line 73 connected to vacuum pump 62. If desired the compaction solvent may be mixed with added light aromatics 54.

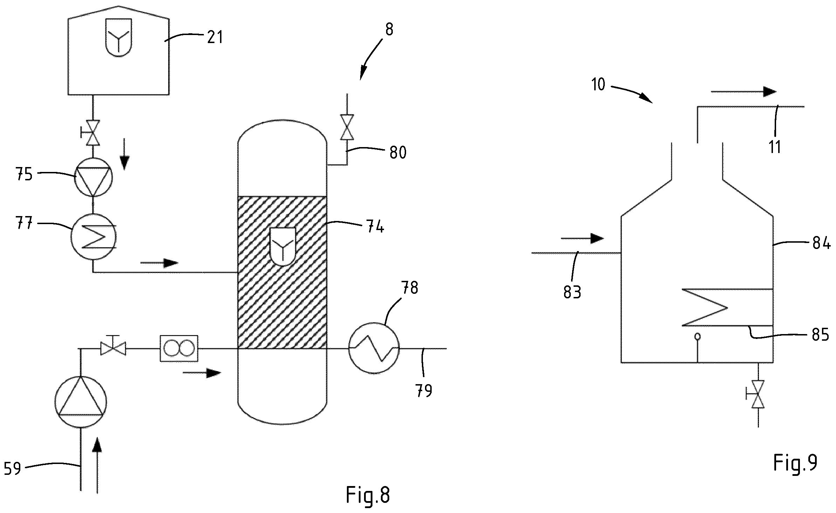

Figure 8 shows in more detail the heating unit 8 for the dehalogenation of polystyrene and/or freonen. The heating unit 8 comprises a reactor 74, such as a plug flow reactor 74. The mixture 59 of polystyrene dissolved in reaction solvent 6 (i.e., here the trimer fraction possibly containing some compaction solvent 16) enters the reactor 74 and is mixed with base 21 supplied over pump 76 and heater 77. The reaction solvent 6 may comprise some compaction solvent 16. The reactor 74 is operated at a dehalogenation temperature of about 250°C and overpressure of about 5 to about 12 bar. The residence time depends on the type and concentration of organohalogen flame retardant in the mixture 59. Generally, the residence time is about 2 min to 60 min. Halogen compound released by flame retardant or freonen is captured by base 21 and halogen salt is formed in the reaction solvent. Any gaseous reaction product formed, such as ammonia may leave the reactor 74 via gas blead 80. The mixture 79 of polystyrene, halogen salt, reaction solvent, and possible remaining reactant such as base 21, and other reaction products, is filtered in a filtration train 81, filtering the mixture 79 at about 100 µm, about 40 µm or 20 µm, and at 1 µm or 0.4 µm. The solids filtered off are collected in sludge tank 82, and may be purified. That filtered liquid 83 enters the pyrolyzation unit 10.

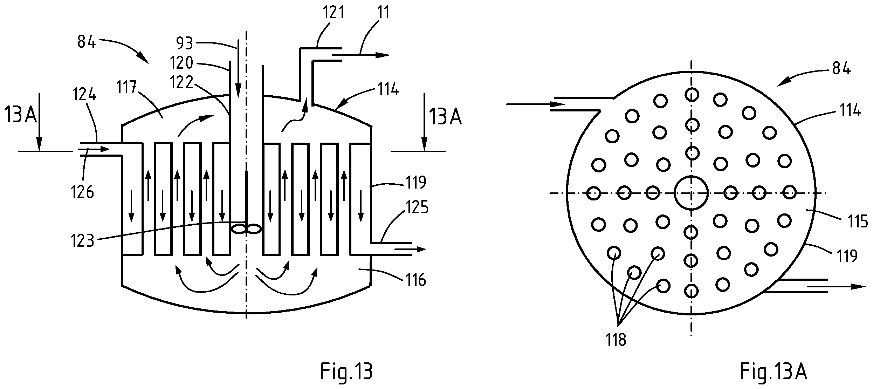

Figure 9 shows the pyrolyzation unit 10 in some more detail. The pyrolyzation reactor 84 is provided with heater 85 for heating the liquid mixture 83 at about 500°C to about 520°C at a pressure of about 1 bar to about 3 bar, to a pyrolyzed mixture 11 comprising depolymerize polystyrene to at least styrene and other depolymerized fractions, such as a dimer fraction and a trimer fraction, and reaction fractions, such as an aliphatic fraction 50 and aromatic fraction 54. A preferred embodiment of the pyrolyzation reaction 84 will be described in Figure 13 below.

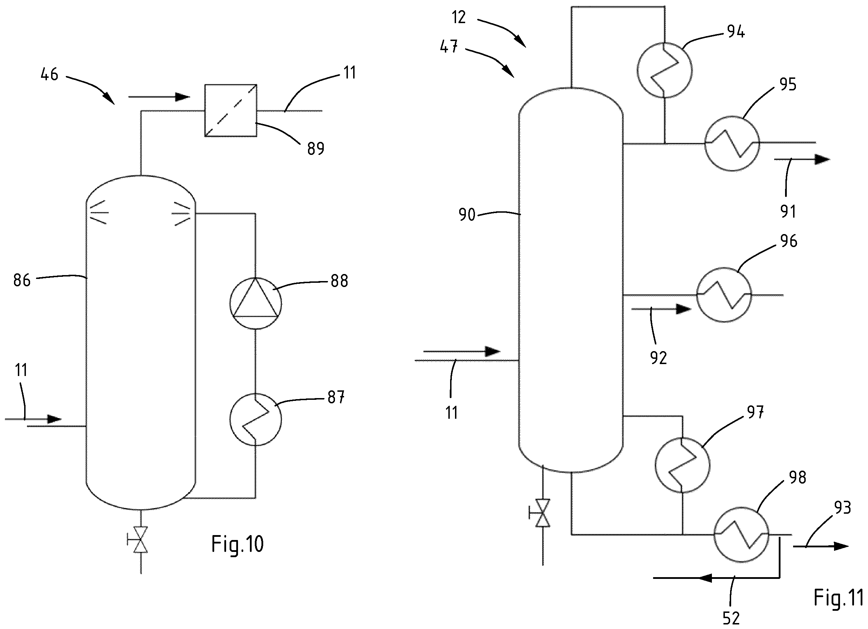

Figure 10 shows in more detail the quencher section 46. It comprises a quencher 86 in which recirculated mixture 11 and/or reaction solvent sprayed after cooling and in cooler 87 and spray pump 88. This is based on the inventive idea in that by using reaction solvent in the form of trimer fraction originating in the process, so that cooling is accomplished without the use of a system-foreign solvent. By cooling the temperature of the mixture 11 is greatly reduced to a temperature at which secondary reactions are substantially avoided or stopped. After quench cooling the mixture passes a filter 89 for removing any remaining or newly formed solids, such as soot and char.

Figure 11 shows in more detail the crude stripper unit 47 of the distillation unit 12. The crude stripper unit 90 distils the pyrolyzed mixture 11 into a top fraction 91, a middle fraction 92 and a bottom fraction 93. Operation is such that the production of top fraction 91 is maximal as it comprises predominantly styrene 13. Top fraction 91 obtained after distillate at the top is cooled in condenser 94 partly recycled and partly cooled in cooler 95 providing cooled top faction 91. Middle fraction 92 is cooled in cooler 96 and further processed in light end stripper unit 48. The bottom fraction 93 is heated in reboiler 97 and partly recycled and partly cooled in cooler 98. This bottom fraction 93 is recycled as the trimer fraction 52 to dissolution unit 4 or stored in storage tank 99 and may be used for generating energy.

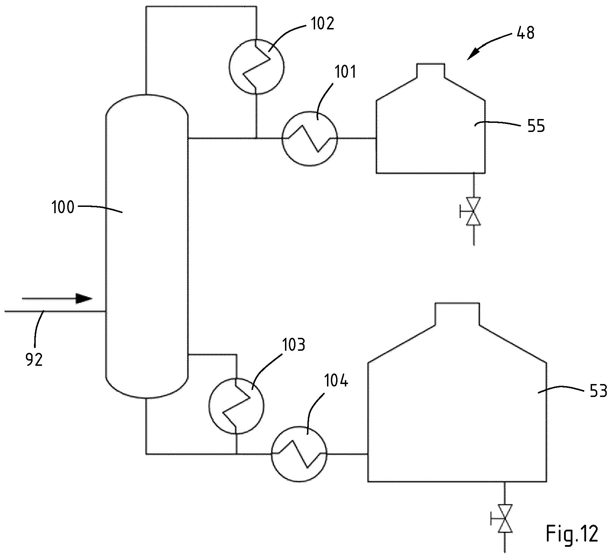

Figure 12 shows in more detail the light end stripper unit 48. Here the middle fraction 92 is stripped in stripper 100 to topdistil off via condenser 101 and cooler 102, light nafta 55 so that at the bottom distils off via reboiler 103 and cooler 104 dimer fraction 53 having the specification of, and/or can be used as compaction solvent 16, and having a flashpoint such that the installation can be safely operated.

Returning to Figure 5, it is shown that top fraction 91 is distilled in distillation column 105 of which the top distillate provides aliphatic solvent 50. The bottom distillate 106 is distilled in distillation column 107 of which the bottom fraction 108 is recycled to distillation column 105. The top distillate 109 is distilled in distillation column 110 providing light aromatics as top fraction 111. The bottom fraction 112 is applied to a styrene distillation column 113 providing high pure styrene 13, and as bottom fraction heavy ends 51.

Finally, Figures 13 and 13A show in more detail an embodiment of reactor 84 in the form of a melt bed reactor 114. The reactor 114 comprises a reactor housing 119 containing a heat exchanger 115 dividing the housing 119 in a bottom section 116 and a top section 117. The bottom section 116 and the top section 117 are connected by plurality of heating pipes 118 which extend over the height of the heat exchanger 115 and are regularly divided over the surface of the heat exchanger. The inlet 120 of the reactor 114 is connected to a downpipe 122 extending coaxially through the heat exchanger 115. The surface area of the downpipe 122 is substantially equal to the surface area of the plurality of heating pipes 118 together. The down pipe 122 opens into the bottom section 116. The top section 117 is connected to the outlet 121. The downpipe 122 may be provided with propellor means 123 for controlling the flow of the mixture 83 through the heat exchanger dependent on the degree of pyrolyzation required. The heat exchanger 115 is provided with a top inlet 124 and a bottom outlet 125 for heating medium 126 which is in counter-current contact with the mixture in the heating pipes. In this manner liquid mixture 93 passes via inlet 120 through the melt bed reactor 144 while being pyrolyzed and leaves the reactor 144 via outlet 121as pyrolyzed mixture 11.

Finally it is noted that the invention extends also to the removal of halogens from organic molecules other than flame retardants and freonen. The inventive concept can also be used for other stable organic molecules like PVC, such as in a mixture of other organic molecules like polymers of pyrolysis mixtures. This with the help of an apolar solvent comprising a strong base at elevated temperatures resulting in a halogen salt, which can be separated easily.

## Claims

1. A method for recycling a polystyrene based material containing organohalogen flame retardant and/or freonen, comprising the steps of:
(i) dissolving the polystyrene based material in a high-boiling, apolar, organic reaction solvent;
(ii) heating the polystyrene contained in the reaction solvent to a temperature so as to release halogen from the flame retardant and/or freonen;
(iii) contacting released halogen with a base so as to form a halogen salt;
(iv) removing the halogen salt;
(v) pyrolyzing polystyrene contained in the reaction solvent at a temperature so as to depolymerize polystyrene; and
(vi) distilling the depolymerized mixture into at least a styrene fraction.

2. The method according to claim 1, wherein the base is added to the reaction solvent so that halogen released in step (ii), is contacted in step (iii) with the base so that the halogen salt is formed in the reaction solvent.

3. The method according to claim 2, wherein the base used for forming the halogen salt in the reaction solvent is selected from the group comprises sodium diamine, potassium tert-butoxide, sodium bis(trimethylsilyl)amide, and P(CH3NCH2CH2)3N.

4. The method according to any of the claims 1-3, wherein the polystyrene based material may comprise up to about 10 wt% foreign polymer, such as polyethylene, polypropylene, and/or polyvinylchloride.

5. The method according to any of the claims 1-4, wherein in step (ii) the polystyrene containing reaction solvent is heated to a temperature in the range of about 150°C to about 350°C, preferably about 180°C° to about 300°C.

6. The method of any of the claims 1-5, wherein the polystyrene to be recycled is a expanded polystyrene, and the method comprises a step of compacting expanded polystyrene in an compaction solvent, whereafter preferably the compaction solvent is at least partly replaced by the reaction solvent, and wherein preferably the compaction solvent is an aromatic compaction solvent, such as a high-boiling distillate fraction obtainable in step (vi) preferably a styrene dimer fraction boiling at about 280°C to about 320°C, or at about 180°C to about 250°C at about 10 to 30 mbar absolute pressure.

7. The method according to claim 6, wherein the compaction solvent is a mixture of the aromatic compaction solvent and an aliphatic compaction solvent, preferably a fraction obtainable in the distillation of step (vi), and wherein preferably the volume ratio of the aromatic compaction solvent and the aliphatic compaction solvent is in the range of about 30:70, preferably about 20:80, more preferable about 10:90.

8. The method according to any of the claims 1-7, wherein the high-boiling apolar organic reaction solvent having a vapor pressure of less than about 10 bar at a temperature in the range of about 250°C to about 450°C, and preferably the reaction solvent is a high-boiling distillate fraction obtainable in step (vi), preferably a styrene trimer fraction boiling at about 380°C to about 420°C.

9. The method according to any of the claims 6-8, wherein the replaced compaction solvent is at least partly recycled to the compaction step.

10. The method of any of the claims 1-9, wherein in step (iv) the halogen salt is removed from the reaction solvent by filtration, centrifugation, cyclonic separation, at a salt particle size of less than about 20 µm, preferably less than about 20 µm, such as less than 1µm, or less than 0.4 µm.

11. The method of any of the claims 1-10, wherein the molar ratio of base to the organohalogen flame retardant and/or freonen is about 1 to about 3, such as about 1.5 to about 2.6.

12. The method of any of the claims 1-11, wherein the pyrolysis of step (v) is carried out at atmospheric pressure at a temperature about 400°C to about 550°C, preferably at about 500°C to about 520°C, or at higher pressure and at corresponding temperatures, and preferably at a pressure of about 1 bar to 3 bar, and preferably the pyrolysis step (v) is carried out in a meltbed reactor.

13. The method of any of the claims 1-12, wherein the removal of organohalogen flame retardant and/or freonen present in the polystyrene based material is at least 90%, preferably at least 95%, more preferably at least 99%.

14. An installation for recycling a polystyrene based material containing a organohalogen flame retardant and/or freonen, such as defined in claims 1-13, comprising:
• a dissolution unit (a) for dissolving the polystyrene based material in a high-boiling, apolar, organic reaction solvent or in a mixture with a high boiling aliphatic reaction solvent;
• a heating unit (b) for heating the polystyrene contained in the reaction solvent to a temperature so as to release halogen from the flame retardant and/or freonen;
• a contacting unit (c) for contacting released halogen with a base so as to form a halogen salt;
• a halogen salt removing unit (d) for removing the halogen salt from the reaction solvent;
• a pyrolyzation unit (e) for pyrolyzing the polystyrene in the reaction solvent at a temperature so as to depolymerize polystyrene; and
• a distillation unit (f) for distilling the depolymerized mixture into at least a styrene fraction.

15. The installation as claimed in claim 14, comprising a compaction unit for compacting expanded polystyrene in a compaction solvent, and for replacing the compaction solvent by the reaction solvent.

16. The installation as claimed in claim 14 or 15, the distillation unit (f) distils the depolymerized mixture to a styrene trimer fraction which is recycled and used at least in apart as reaction solvent, and/or the distillation unit (f) distils the depolymerized mixture to a styrene dimer fraction which is recycled and is used at least in apart as compaction solvent for expanded polystyrene.

17. The installation of any of the claims 14-16, wherein the pyrolyzation unit (e) comprises a meltbed reactor.

18. The method of any of the claims 1-13, or the installation of any of the claims 14-17,wherein the organohalogen flame retardant is an organobromo, organochloro or organofluoro flame retardant, such as hexabromocyclododecane (HBCD), tetrabromobisphenol A (2,3-Dibromopropyl)ether (BDDP or FR-720), tris(tribromophenoxy) triazine (FR-245), Tetrabromobisphenol-A-bis(2,3-dibromo-2-methylpropylether), 1,1'-(isopropylidene)bis[3,5-dibromo-4-(2,3-dibromo-2-methylpropoxy)benzene] ( TBBPA-DBMPE or AP 1300 SF), *Tris*(1-chloro-2-propyl) phosphate (TCPP), tetradecafluorohexane (TDFH), and the freonen comprise hydrochlorofluorocarbon (HCFC, such as R-22, R-12 and R134a).

19. The method of any of the claims 1-13 and 18, or the installation of any of the claims 14-18, wherein the polystyrene based material comprises polystyrene, expanded polystyrene (EPS), extruded polystyrene (XPS), such as used in packaging foils and containers, styrene-butadiene rubber (SBR), acrylonitrile-butadiene-styrene (ABS).

## Patentansprüche

1. Verfahren zum Recyceln eines Materials auf Polystyrolbasis, das Organohalogenflammschutzmittel und/oder Freonen enthält, umfassend die Schritte:
(i) Auflösen des Materials auf Polystyrolbasis in einem hochsiedenden, apolaren, organischen Reaktionslösungsmittel;
(ii) Erhitzen des Polystyrols, das in dem Reaktionslösungsmittel enthalten ist, auf eine Temperatur, um Halogen aus dem Flammschutzmittel und/oder Freonen freizusetzen;
(iii) Kontaktieren eines freigesetzten Halogens mit einer Base, um ein Halogensalz auszubilden;
(iv) Entfernen des Halogensalzes;
(v) Pyrolysieren des Polystyrols, das in dem Reaktionslösungsmittel enthalten ist, bei einer Temperatur, um das Polystyrol zu depolymerisieren; und
(vi) Destillieren der depolymerisierten Mischung in mindestens eine Styrolfraktion.

2. Verfahren nach Anspruch 1, wobei die Base dem Reaktionslösungsmittel zugegeben wird, sodass das Halogen, das in Schritt (ii) freigesetzt wird, in Schritt (iii) mit der Base in Kontakt gebracht wird, so dass das Halogensalz in dem Reaktionslösungsmittel ausgebildet wird.

3. Verfahren nach Anspruch 2, wobei die Base, die zum Ausbilden des Halogensalzes in dem Reaktionslösungsmittel verwendet wird, aus der Gruppe ausgewählt ist, die Natriumdiamin, Kalium-tert-butoxid, Natriumbis(trimethylsilyl)amid und P(CH3NCH2CH2)3N umfasst.

4. Verfahren nach einem der Ansprüche 1 bis 3, wobei das Material auf Polystyrolbasis bis zu etwa 10 Gew.-% Fremdpolymer wie Polyethylen, Polypropylen und/oder Polyvinylchlorid umfassen kann.

5. Verfahren nach einem der Ansprüche 1 bis 4, wobei in Schritt (ii) das Polystyrol enthaltende Reaktionslösungsmittel auf eine Temperatur in dem Bereich von etwa 150 °C bis etwa 350 °C, vorzugsweise etwa 180 °C bis etwa 300 °C, erhitzt wird.

6. Verfahren nach einem der Ansprüche 1 bis 5, wobei das zu recycelnde Polystyrol ein expandiertes Polystyrol ist und das Verfahren einen Schritt eines Verdichtens von expandiertem Polystyrol in einem Verdichtungslösungsmittel umfasst, wonach vorzugsweise das Verdichtungslösungsmittel mindestens teilweise durch das Reaktionslösungsmittel ersetzt wird, und wobei vorzugsweise das Verdichtungslösungsmittel ein aromatisches Verdichtungslösungsmittel ist, wie eine hochsiedende Destillatfraktion, die in Schritt (vi) erhältlich ist, vorzugsweise eine Styroldimerfraktion, die bei etwa 280 °C bis etwa 320 °C oder bei etwa 180 °C bis etwa 250 °C bei etwa 10 bis 30 mbar absolutem Druck siedet.

7. Verfahren nach Anspruch 6, wobei das Verdichtungslösungsmittel eine Mischung aus dem aromatischen Verdichtungslösungsmittel und einem aliphatischen Verdichtungslösungsmittel ist, vorzugsweise eine Fraktion, die bei der Destillation von Schritt (vi) erhältlich ist, und wobei vorzugsweise das Volumenverhältnis des aromatischen Verdichtungslösungsmittels und des aliphatischen Verdichtungslösungsmittels in dem Bereich von etwa 30 : 70, vorzugsweise etwa 20 : 80, mehr bevorzugt etwa 10 : 90 liegt.

8. Verfahren nach einem der Ansprüche 1 bis 7, wobei das hochsiedende, apolare organische Reaktionslösungsmittel einen Dampfdruck von weniger als etwa 10 Bar bei einer Temperatur in dem Bereich von etwa 250 °C bis etwa 450 °C aufweist, und vorzugsweise wobei das Reaktionslösungsmittel eine hochsiedende Destillatfraktion ist, die in Schritt (vi) erhältlich ist, vorzugsweise eine Styroltrimerfraktion, die bei etwa 380 °C bis etwa 420 °C siedet.

9. Verfahren nach einem der Ansprüche 6 bis 8, wobei das ersetzte Verdichtungslösungsmittel mindestens teilweise in den Verdichtungsschritt zurückgeführt wird.

10. Verfahren nach einem der Ansprüche 1 bis 9, wobei in Schritt (iv) das Halogensalz durch Filtration, Zentrifugation oder Zyklontrennung aus dem Reaktionslösungsmittel entfernt wird, bei einer Salzpartikelgröße von weniger als etwa 20 µm, vorzugsweise weniger als etwa 20 µm, wie weniger als 1 µm oder weniger als 0,4 µm.

11. Verfahren nach einem der Ansprüche 1 bis 10, wobei das Molverhältnis der Base zu dem Organohalogenflammschutzmittel und/oder den Freonen etwa 1 bis etwa 3, wie etwa 1,5 bis etwa 2,6, beträgt.

12. Verfahren nach einem der Ansprüche 1 bis 11, wobei die Pyrolyse in Schritt (v) bei Luftdruck bei einer Temperatur von etwa 400 °C bis etwa 550 °C, vorzugsweise bei etwa 500 °C bis etwa 520 °C, oder bei höherem Druck und bei entsprechenden Temperaturen und vorzugsweise bei einem Druck von etwa 1 Bar bis 3 Bar durchgeführt wird, und vorzugsweise wobei der Pyrolyseschritt (v) in einem Schmelzbettreaktor durchgeführt wird.

13. Verfahren nach einem der Ansprüche 1 bis 12, wobei die Entfernung des Organohalogenflammschutzmittels und/oder der Freonen, das/die in dem Material auf Polystyrolbasis vorhanden sind, mindestens 90 %, vorzugsweise mindestens 95 %, noch besser mindestens 99 % beträgt.

14. Anlage zum Recyceln eines Materials auf Polystyrolbasis, das ein Organohalogenflammschutzmittel und/oder Freonen enthält, wie in den Ansprüchen 1 bis 13 definiert, umfassend:
• eine Auflösungseinheit (a) zum Auflösen des Materials auf Polystyrolbasis in einem hochsiedenden, unpolaren, organischen Reaktionslösungsmittel oder in einer Mischung mit einem hochsiedenden aliphatischen Reaktionslösungsmittel;
• eine Heizeinheit (b) zum Erhitzen des Polystyrols, das in dem Reaktionslösungsmittel enthalten ist, auf eine Temperatur, um Halogen aus dem Flammschutzmittel und/oder den Freonen freizusetzen;
• eine Kontakteinheit (c) zum Kontaktieren des freigesetzten Halogens mit einer Base, um ein Halogensalz auszubilden;
• eine Halogensalzentfernungseinheit (d) zum Entfernen des Halogensalzes aus dem Reaktionslösungsmittel;
• eine Pyrolyseeinheit (e) zum Pyrolysieren des Polystyrols in dem Reaktionslösungsmittel bei einer Temperatur, um Polystyrol zu depolymerisieren; und
• eine Destillationseinheit (f) zum Destillieren der depolymerisierten Mischung in mindestens eine Styrolfraktion.

15. Anlage nach Anspruch 14, umfassend eine Verdichtungseinheit zum Verdichten von expandiertem Polystyrol in einem Verdichtungslösungsmittel und zum Ersetzen des Verdichtungslösungsmittels durch das Reaktionslösungsmittel.

16. Anlage nach Anspruch 14 oder 15, wobei die Destillationseinheit (f) die depolymerisierte Mischung zu einer Styroltrimerfraktion destilliert, die zurückgeführt und mindestens teilweise als Reaktionslösungsmittel verwendet wird, und/oder die Destillationseinheit (f) die depolymerisierte Mischung zu einer Styroldimerfraktion destilliert, die zurückgeführt und mindestens teilweise als Verdichtungslösungsmittel für expandiertes Polystyrol verwendet wird.

17. Anlage nach einem der Ansprüche 14 bis 16, wobei die Pyrolyseeinheit (e) einen Schmelzbettreaktor umfasst.

18. Verfahren nach einem der Ansprüche 1 bis 13 oder Anlage nach einem der Ansprüche 14 bis 17, wobei das Organohalogenflammschutzmittel ein Organobrom-, Organochlor- oder Organofluorflammschutzmittel, wie Hexabromcyclododecan (HBCD), Tetrabrombisphenol-A-(2,3-dibrompropyl)ether (BDDP oder FR-720), Tris(tribromphenoxy)triazin (FR-245), Tetrabrombisphenol-A-bis(2,3-dibrom-2-methylpropylether), 1,1'-(Isopropyliden)bis[3,5-dibrom-4-(2,3-dibrom-2-methylpropoxy)benzol] (TBBPA-DBMPE oder AP 1300 SF), *Tris*(1-chlor-2-propyl)phosphat (TCPP), Tetradecafluorhexan (TDFH), ist, und die Freone Fluorchlorkohlenwasserstoffe (H-FCKW, wie R-22, R-12 und R134a) umfassen.

19. Verfahren nach einem der Ansprüche 1 bis 13 und 18 oder Anlage nach einem der Ansprüche 14 bis 18, wobei das Material auf Polystyrolbasis Polystyrol, expandiertes Polystyrol (EPS), extrudiertes Polystyrol (XPS), wie es in Verpackungsfolien und -behältern verwendet wird, Styrol-Butadien-Kautschuk (SBR), Acrylnitril-Butadien-Styrol (ABS) umfasst.

## Revendications

1. Procédé de recyclage d'un matériau à base de polystyrène contenant un retardateur de flamme organohalogéné et/ou du fréon, comprenant les étapes consistant à :
(i) dissoudre le matériau à base de polystyrène dans un solvant de réaction organique apolaire à point d'ébullition élevé ;
(ii) chauffer le polystyrène contenu dans le solvant de réaction à une température permettant de libérer l'halogène de l'agent ignifuge et/ou du fréon ;
(iii) mettre en contact l'halogène libéré avec une base de manière à former un sel d'halogène ;
(iv) éliminer le sel d'halogène ;
(v) pyrolyser le polystyrène contenu dans le solvant de réaction à une température permettant de dépolymériser le polystyrène ; et
(vi) distiller le mélange dépolymérisé en au moins une fraction de styrène.

2. Procédé selon la revendication 1, dans lequel la base est ajoutée au solvant de réaction de sorte que l'halogène libéré à l'étape (ii) est mis en contact à l'étape (iii) avec la base de sorte que le sel d'halogène se forme dans le solvant de réaction.

3. Procédé selon la revendication 2, dans lequel la base utilisée pour former le sel d'halogène dans le solvant de réaction est choisie parmi le groupe comprenant la diamine de sodium, le tert-butoxyde de potassium, le bis(triméthylsilyl)amide de sodium, et P(CH3NCH2CH2)3N.

4. Procédé selon l'une quelconque des revendications 1 à 3, dans lequel le matériau à base de polystyrène peut comprendre jusqu'à environ 10 % en poids de polymère étranger, tel que le polyéthylène, le polypropylène et/ou le polychlorure de vinyle.

5. Procédé selon l'une quelconque des revendications 1 à 4, dans lequel, à l'étape (ii), le solvant de réaction contenant du polystyrène est chauffé à une température comprise entre 150 °C et 350 °C environ, de préférence entre 180 °C et 300 °C environ.

6. Procédé selon l'une quelconque des revendications 1 à 5, dans lequel le polystyrène à recycler est un polystyrène expansé, et le procédé comprend une étape de compactage du polystyrène expansé dans un solvant de compactage, après quoi, de préférence, le solvant de compactage est au moins partiellement remplacé par le solvant de réaction, et dans lequel, de préférence, le solvant de compactage est un solvant de compactage aromatique, tel qu'une fraction de distillat à point d'ébullition élevé obtenue à l'étape (vi), de préférence une fraction de dimère de styrène dont le point d'ébullition se situe entre 280 °C et 320 °C environ, ou entre 180 °C et 250 °C environ, à une pression absolue comprise entre 10 et 30 mbar environ.

7. Procédé selon la revendication 6, dans lequel le solvant de compactage est un mélange de solvant de compactage aromatique et de solvant de compactage aliphatique, de préférence une fraction pouvant être obtenue lors de la distillation de l'étape (vi), et dans lequel le rapport de volume entre le solvant de compactage aromatique et le solvant de compactage aliphatique est d'environ 30:70, de préférence d'environ 20:80, plus préférablement d'environ 10:90.

8. Procédé selon l'une quelconque des revendications 1 à 7, dans lequel le solvant de réaction organique apolaire à point d'ébullition élevé a une pression de vapeur inférieure à environ 10 bars à une température comprise entre environ 250 °C et environ 450 °C, et de préférence le solvant de réaction est une fraction de distillat à point d'ébullition élevé obtenue à l'étape (vi), de préférence une fraction de trimère de styrène dont le point d'ébullition est compris entre environ 380 °C et environ 420 °C.

9. Procédé selon l'une quelconque des revendications 6 à 8, dans lequel le solvant de compactage remplacé est au moins partiellement recyclé à l'étape de compactage.

10. Procédé selon l'une quelconque des revendications 1 à 9, dans lequel, à l'étape (iv), le sel halogène est éliminé du solvant de réaction par filtration, centrifugation, séparation cyclonique, à une taille de particule de sel inférieure à environ 20 µm, de préférence inférieure à environ 20 µm, telle qu'inférieure à 1 µm, ou inférieure à 0,4 µm.

11. Procédé selon l'une quelconque des revendications 1 à 10, dans lequel le rapport molaire entre la base et le retardateur de flamme organohalogéné et/ou le fréon est d'environ 1 à environ 3, tel qu'environ 1,5 à environ 2,6.

12. Procédé selon l'une quelconque des revendications 1 à 11, dans lequel la pyrolyse de l'étape (v) est effectuée à la pression atmosphérique à une température d'environ 400 °C à environ 550 °C, de préférence d'environ 500 °C à environ 520 °C, ou à une pression plus élevée et à des températures correspondantes, et de préférence à une pression d'environ 1 bar à 3 bars, et de préférence l'étape de pyrolyse (v) est effectuée dans un réacteur à lit de fusion.

13. Procédé selon l'une quelconque des revendications 1 à 12, dans lequel l'élimination des retardateurs de flamme organohalogénés et/ou du fréon présents dans le matériau à base de polystyrène est d'au moins 90 %, de préférence d'au moins 95 %, plus préférablement d'au moins 99 %.

14. Installation de recyclage d'un matériau à base de polystyrène contenant un retardateur de flamme organohalogéné et/ou du fréon, telle que définie dans les revendications 1 à 13, comprenant :
• une unité de dissolution (a) pour dissoudre le matériau à base de polystyrène dans un solvant de réaction organique apolaire à point d'ébullition élevé ou dans un mélange avec un solvant de réaction aliphatique à point d'ébullition élevé ;
• une unité de chauffage (b) pour chauffer le polystyrène contenu dans le solvant de réaction à une température permettant de libérer l'halogène de l'agent ignifuge et/ou du fréonen ;
• une unité de mise en contact (c) pour mettre en contact l'halogène libéré avec une base afin de former un sel d'halogène ;
• une unité d'élimination des sels halogènes (d) pour éliminer les sels halogènes du solvant de réaction ;
• une unité de pyrolyse (e) pour pyrolyser le polystyrène dans le solvant de réaction à une température permettant de dépolymériser le polystyrène ; et
• une unité de distillation (f) pour distiller le mélange dépolymérisé en au moins une fraction de styrène.

15. Installation selon la revendication 14, comprenant une unité de compactage pour compacter le polystyrène expansé dans un solvant de compactage, et pour remplacer le solvant de compactage par le solvant de réaction.

16. Installation selon la revendication 14 ou 15, l'unité de distillation (f) distille le mélange dépolymérisé en une fraction de trimère de styrène qui est recyclée et utilisée au moins en partie comme solvant de réaction, et/ou l'unité de distillation (f) distille le mélange dépolymérisé en une fraction de dimère de styrène qui est recyclée et utilisée au moins en partie comme solvant de compactage pour le polystyrène expansé.

17. Installation selon l'une quelconque des revendications 14 à 16, dans laquelle l'unité de pyrolyse (e) comprend un réacteur à lit de fusion.

18. Procédé selon l'une quelconque des revendications 1 à 13, ou installation selon l'une quelconque des revendications 14 à 17, dans lequel/laquelle le retardateur de flamme organohalogéné est un retardateur de flamme organobromé, organochloré ou organofluoré, tel que l'hexabromocyclododécane (HBCD), le tétrabromobisphénol A (2,3-Dibromopropyl)éther (BDDP ou FR-720), la tris(tribromophénoxy) triazine (FR-245), le Tétrabromobisphénol-A-bis(2,3 - dibromo-2-méthylpropyléther), 1,1'-(isopropylidène)bis[3,5-dibromo-4-(2,3-dibromo-2-méthylpropoxy)benzène] ( TBBPA-DBMPE ou AP 1300 SF), Phosphate de *tris*(1chloro-2-propyle) (TCPP), tétradécafluorohexane (TDFH), et les fréons comprennent les hydrochlorofluorocarbones (HCFC, tels que R-22, R-12 et R134a).

19. Procédé selon l'une quelconque des revendications 1 à 13 et 18, ou installation selon l'une quelconque des revendications 14 à 18, dans lequel/laquelle le matériau à base de polystyrène comprend du polystyrène, du polystyrène expansé (EPS), du polystyrène extrudé (XPS), tel qu'il est utilisé dans les feuilles d'emballage et les conteneurs, du caoutchouc styrène-butadiène (SBR), de l'acrylonitrile-butadiène-styrène (ABS).
